# EUROPEAN PATENT APPLICATION

(11) **EP 2 186 517 A1**
(43) Date of publication of application: **19.05.2010**
(21) Application number: 08805375.6
(22) Date of filing: 07.08.2008
(51) Int. Cl.: A61K 31/5517, A61K 31/415, A61P 25/30

(54) **USE AND METHODS OF USE FOR AN ANTAGONIST OF THE SEROTIN3 RECEPTOR (5-HT3) AND A SELECTIVE MODULATOR OF CHLORIDE CHANNELS FOR THE TREATMENT OF ADDICTION TO OR DEPENDENCE ON MEDICINES/DRUGS OR NERVOUS SYSTEM DISORDERS**

(30) Priority: 08.08.2007 US 963957 P
(71) Applicant: Legarda Ibañez, Juan José, 28035 Madrid (ES)
(72) Inventor: Legarda Ibañez, Juan José, 28035 Madrid (ES)
(74) Representative: Maldonado Jordan, Julia
(86) International application number: PCT/ES2008/000552
(87) International publication number: WO 2009/027564

(57) **Abstract**

The present invention relates to the use and methods of using a combination of a serotonin, (5-HT₃) receptor antagonist, such as ondansetron, and a selective chloride channel modulator, such as flumazenil, for treatment of patients with drug addiction or dependence. Examples of the drugs include prescription drugs as well as illegal drugs, such as, but not limited to, alcohol, cocaine, nicotine, marijuana, benzodiazepines and opiates. Furthermore, this combination can be used for treatment of patients suffering from central nervous system (CNS) disorders such as, but not limited to, depression, emotional and mood disorders and loss of memory.

## Description

### Field of the Invention

The present invention relates to the use and methods of using a combination of a seroronin₃ (5-HT₃) receptor antagonist, such as ondansetron, and a selective chloride channel modulator, such as flumazenil, for treatment of patients with drug addiction or dependence. Examples of drugs include prescription drugs as well as illegal drugs, such as, but not limited to, alcohol, cocaine, nicotine, marijuana, benzodiazepines and opiates. Furthermore, this combination can be used for treatment of patients suffering from central nervous system (CNS) disorders such as, but not limited to, depression, mood and emotional disorders and loss of memory..

The present invention also relates to the use of said combination comprising a therapeutically effective amount of a serotonin₃ (5-HT₂) receptor antagonist and a therapeutically effective amount of a selective chloride channel modulator for preparing a medicament for treatment of drug addiction or dependence or for treatment of a central nervous system (CNS) disorder.

### Background of the Invention

Since 1990 there has been a sharp rise in the abuse and addiction of controlled prescription drugs such as opioids, central nervous system (CNS) depressants, CNS stimulants (Trescot, Boswell *et al*. 2006). Prescription drugs are now the fourth most abused substances in the United States.

Addiction is referred to individuals who continue to use a given drug despite their own best interest. However, pharmacological addiction is referred to the use of a tolerance-inducing drug in sufficient amount to cause tolerance to and physical dependence on that drug. Tolerance represents an adaptation to repeated exposure to a drug such that the pharmacological response is diminished. Physical dependence is a state manifested by withdrawal symptoms when drug intake is terminated or significantly reduced (O'Brien 1997). Drug addiction is an issue with many consequences, one of them being, addiction withdrawal symptoms experienced when the drug or drugs are discontinued. These symptoms of drug withdrawal at times may be severe, if not life threatening. Drug withdrawal has many variables. Everything from the type of drug, amount of regular use, to the length of time the drug was abused, factors into how intense or mild an individuals drug withdrawal experience will be. As mentioned, the type of drug used plays an important part in determining the length and severity of drug withdrawal.

Another factor to be aware of during withdrawal is drug craving. Drug craving is the result of the drugs imprinting in the memory, a pleasant association of euphoria with the drug. The subconscious memory then motivates the individual to seek this drug because of the false imprint. The brain, in effect, has been trained that using the drug is the fastest way to feel good. Due to the extreme physiological or physical pain some experience during drug withdrawal they can relapse before they complete the withdrawal process.

Symptoms of withdrawal from either illegal drugs or prescription medicaments depend on the drug or combination of drugs. Common withdrawal symptoms include: abdominal pain, nausea and vomiting, drenching sweats, nervousness and shaking or seizures (Ref: http://www.webmd.com/hw/healthguide atoz/tv5810.asp)

There are only three drugs approved by the US Food and Drug Administration (FDA) for the treatment of alcoholism and the feasibility of pharmacological treatment in alcoholism has been demonstrated by these 3 currently approved medicaments, the aldehyde dehydrogenase blocker disulfiram, the opioid antagonist naltrexone and the functional glutamate antagonist acamprosate. However, several drugs like nalmafene, topiramate, and ondansetron that are approved for other indications are being used experimentally to treat people with alcohol dependence and abstinence (Collins, McAllister *et al*. 2006). Early human efficacy data are available for the 5HT₃ antagonist ondansetron, the GABA-B antagonist baclofen and the anticonvulsant topiramate and efficacy studied on a battery of animal models has accumulated sufficient preclinical validation to merit clinical development of drugs acting on the cannabinoid CB1 receptor, receptors modulating glutamatergic transmission (mGluR2, 3 and 5), and receptors for stress-related neuropeptides corticotropin releasing factor (CRF), neuropeptide Y (NPY) and nociceptin (Heilig and Egli 2006).

### Alcohol

Ondansetron (GR38032F, CAS 103639-04-9) chemically is (±)1,3,3,9-tetrahydro-9-methyl-3-[(2-methyl-1H-imidazol-1-yl)methyl]-4H-carbazol-4-one, monohydrochlororide, dehydrate with a molecular weight of 365.9. This racemic form of ondansetron is a white to off-white powder that is soluble in water and normal saline and a selective blocking agent of the serotonin 5-HT₃ receptor type. Ondansetron is an antiemetic producing its effect by blocking 5-HT₃ receptors in the brain and periphery. While ondansetron's mechanism of action for its antiemetic action is not fully characterized, it is not a dopamine receptor antagonist. Serotonin receptors of 5-HT₃ type are present both peripherally on the vagal nerve terminals and centrally in the chemoreceptor trigger zone of the area posterma. It is not certain ondansteron's antiemetic action in chemotherapy induced emesis is mediated centrally, peripherally or both. It is mostly used for the management of nausea and vomiting induced by cytotoxic chemotherapy and radiotherapy, and for the prevention and treatment of postoperative nausea and vomiting.

It has been proposed that 5-HT₃ receptor antagonist ondansetron, can be used as an anxiolytic agent and that ondansetron may have major therapeutic advantages over currently available anxiolytic agents (Costall, Jones *et al.* 1989). In experimental studies ondansetron (0.1 and 1 mg/kg PO) increased the number of shocks taken by mice in punished exploratory behavior in the four-plate test (Dooley and Klamt 1993). In a nonpunishing quantitative model of anxiety ondansetron in all the doses tested (0.01, 0.1 and 1 mg/kg i.p.) showed significant anxiolytic action compared to naive mice, but it was less potent as compared to a well-known anxiolytic, diazepam (1 mg/kg) (Roychoudhury and Kulkarni 1997). The effects of anxiogenic and anxiolytic compounds on the electric self-stimulation of the medial fore-brain bundle in male rats were studied to find out if there is a link between reward and anxiety-related behaviors. The 5-HT₃ antagonist ondansetron and a benzodiazepine receptor antagonist flumazenil did not cause any substantial changes of the self-stimulation (Borisenko, Meng *et al.* 1996). These studies indicated that ondansetron has anxiolytic action but does not affect reward or has any major effect on self-stimulation. Evaluation of anxiolytic action of ondansetron in rats during withdrawal from chronic chlordiazepoxide showed mixed results (Rezazadeh, Prather *et al.* 1992). Experiments were conducted to determine the efficacy of ondansetron in reversing various aspects of benzodiazepine withdrawal in rats. Three tests were used in which the benzodiazepine antagonist flumazenil was administered to rats receiving chronic administration of chlordiazepoxide. Inconsistent results were obtained and it was concluded that ondansetron is not likely to have any efficacy in humans for the treatment of sedative-hypnotic withdrawal (Prather, Rezazadeh *et al*. 1993).

VA21B7 (3-[2-(4'-piperynylpiperazinyl)indplyl] carboxaldehyde) was synthesized as a potential 5-HT₃ receptor antagonist and it was found that VA21B7 was also able to release suppressed behavior in the punished-drinking test (Artaiz, Romero *et al*. 1995). Ondansetron, 5-HT₃ receptor antagonist, has been shown to be an effective treatment for early-onset alcoholism. Ondansetron (16 microg/kg twice daily) showed greater therapeutic efficacy at alleviating symptoms of overall mood disturbance, fatigue, vigor, confusion/bewilderment, and depression among early-onset alcoholism compared with late-onset alcoholism. Ondansetron's ability to improve symptoms of depression, anxiety, and hostility among early-onset alcoholics may make an additional contribution to its therapeutic effect (Johnson, Ait-Daoud *et al.* 2003). An 8-week, prospective, open-label study of ondansetron (4 microg/kg b.i.d.) in 12 adolescents who had alcohol dependence study was conducted and it was found that oral ondansetron was safe and well tolerated and a significant decrease for drinks per day was observed (Dawes, Johnson et al 2005). It was further observed that ondansetron diminished drinking in adolescents with alcohol dependence through a reduction in "craving" as measured by the Adolescent Obsessive-Compulsive Drinking Scale (Dawes, Johnson *et al*. 2005). In another study, a cohort of 253 out of 321 enrolled alcohol dependent subjects was entered into a 1-week lead-in single-blind placebo period followed by 11 weeks of double-blind outpatient treatment. Study design was a 2 (early-onset alcoholics versus late-onset alcoholics) x 4 medication dose (placebo, or ondansetron 1, 4, or 16 microg/kg b.i.d) x 13 (visits) factorial analysis of variance. Craving was measured at each visit using seven visual analogue scales. Ondansetron 4 microg/kg b.i.d. reduced overall craving significantly among early-onset alcoholics. Decreased overall craving was positively correlated with reduced drinking and negatively associated with increased abstinence. Compared with placebo, ondansetron (4 microg/kg b.i.d.) was associated with significant reductions in overall craving in early-onset alcoholics but not late-onset alcoholics (Johnson, Roache *et al*. 2002).

Involvement of benzodiazepine-GABA complex has been proposed in alcohol addiction. A 50% reduction in electroenoephalography measured sleep time was seen in the alcohol dependent group despite the same degree of occupancy by midazolam as seen in the control group. This suggests that alcohol dependence in man is associated with a reduced EEG sleep response to the benzodiazepine agonist, midazolam, which is not explained by reduced benzodiazepine receptor occupancy (Lingford-Hughes, Wilson *et al*. 2005). Prolonged exposure to ethanol produces tolerance and dependence and its withdrawal alters GABA(A) receptor subunit gene expression and function.

Flumazenil (Ro 15-1788, CAS 78755-81-4) is a benzodiazepine receptor antagonist, which belongs to the class of selective chloride modulators. Chemically flumazenil is ethyl 8-fluoro-5,6-dihydro-5-methyl-6-oxo-4H-imidazo[1,5-a](1,4)benzodiazepine-3-carboxylate. It has an imidazobenzodiazepine structure, a molecular weight of 303.3. Flumazenil is a white to off-white crystalline compound with an octanol:buffer partition coefficient of 14 to 1 at pH 7.4. It is insoluble in water but slightly soluble in acidic aqueous solutions. Flumazenil selectively prevented the up-regulation of alpha(4)-subunit gene expression and an associated change and the increase in its own modulatory action (Biggio, Gorini *et al.* 2007). Effect of flumazenil on ethanol withdrawal syndrome has been investigated in rats and it was found that certain actions of flumazenil on the experimental ethanol withdrawal syndrome may suggest a potential beneficial effect of this drug in the treatment of ethanol withdrawal syndrome in alcoholics (Stanford and Stanford 1999). It was also found that high-affinity benzodiazepine antagonists reduce responding maintained by ethanol presentation in ethanol-preferring rats (Jure, Zuccarelli *et al*. 1998). Flumazenil did not produce any anxiolytic effects in rat studies using an elevated plus maze test. However, withdrawal from ethanol treatment produced a decrease in open arm entries and flumazenil blocked these changes (Moy, Knapp *et al.* 1997). Continuous flumazenil infusion has been previously used in the treatment of zolpidem (Ambien) and ethanol coingestion (Burton, Lyon *et al*. 1998). In a 15 alcohol-dependent subjects double-blind placebo-controlled cross-over study it was found that flumazenil was neither anxiolytic nor anxiogenic although withdrawal scores were reduced during the course of the study (Potokar, Coupland *et al*. 1997).

Due to the propensity for naltrexone (a mu opioid antagonist) to reduce alcohol's rewarding effects and the added knowledge that 5-HT₃ receptors may themselves mediate alcohol reward via activation of the endogenous opioid system, it was thought that the combination of ondansetron and naltrexone would act synergistically and would be an effective treatment in early-onset alcoholics. It was found that combining ondansetron and naltrexone effectively treats biologically predisposed alcoholics (Johnson, Ait-Daoud *et al*. 2000). In another study, a combination of ondansetron and naltrexone (a mu opioid receptor antagonist) appears to act synergistically at improving the drinking outcomes of early onset alcoholics. This combination has been found to reduce craving and automaticity of drinking (Ait-Daoud, Johnson, *et al*. 2001). Studies support the suggestion that multiple neuronal systems contribute to the ethanol withdrawal sign of decreased social interaction (Knapp, Overstreet *et al*. 2004) which support our hypothesis of using a combination of drugs for the treatment of alcohol withdrawal.

There is no study describing the use of a combination of ondansetron and flumazenil for the management of alcohol addiction or dependence. The use of both ondansetron and flumazenil either in the form a combined formulation or administered according to a protocol is proposed for the first time. It was found that this combination can be very useful in the management of alcohol addiction or dependence because combination of ondansetron and flumazenil markedly improves results compared to use of ondansetron or flumazenil alone. The use of a combination of ondansetron and flumazenil is novel.

### Cocaine

Ondansetron inhibited cocaine self-administration and sensitization regardless of whether given after each second cocaine regimen dose or during the second withdrawal period indicating that ondansetron may be a useful treatment for cocaine addicts who have undergone previous sensitization periods (Davidson, Lee *et al*. 2002). Ondansetron, given 3.5 h after intravenous cocaine self-administration, has been found to attenuate self-administration the following day, however, when administered 30 min before the cocaine session had no effect on cocaine intake the following day. Ondansetron may be an effective cocaine abuse therapy when administered during acute cocaine withdrawal period (Davidson, Lazarus *et al.* 2004). In a pilot randomized, double-blind, 10-week controlled trial, 63 treatment-seeking, cocaine-dependent men and women received ondansetron (0.25 mg, 1.0 mg, or 4.0 mg twice daily) or placebo. Ondansetron was well tolerated, causing no serious adverse events. The ondansetron 4.0 mg group had the lowest dropout rate among all treatment groups and a greater rate of improvement in percentage of participants with a cocaine-free week compared with the placebo group (p = 0.02) (Johnson, Roache *et al*. 2006). Blockade of cocaine sensitization and tolerance by the co-administration of ondansetron, a 5-HT₃ receptor antagonist, and cocaine has been Observed. Daily injections of ondansetron had no consistent or significant effect on the subsequent behavioral response to cocaine in the saline control subjects. In contrast, daily injections of ondansetron with cocaine significantly blocked the development of sensitization with an inverted U-shape dose-response curve. In the continuous cocaine group ondansetron injections also attenuated the development of behavioral tolerance. The results therefore indicate that 5-HT₃ receptor stimulation during continuous and intermittent cocaine administration is an important link in the development of behavioral tolerance and sensitization (King, Xiong *et al*. 1997) and blockade of the expression of sensitization and tolerance by ondansetron administered during withdrawal from intermittent and continuous cocaine has been observed (King, Xiong *et al*. 1998).

In a study conducted in rats flumazenil was found to unmask seizures and increase the incidence of death in the group receiving combined cocaine-diazepam compared to cocaine alone or diazepam alone (Derlet and Albertson 1994).

There is no study describing the use of a combination of ondansetron and flumazenil for the management of cocaine addiction or dependence. The use of both ondansetron and flumazenil either in the form a combined formulation or administered according to a protocol for the management of cocaine addiction or dependence is proposed for the first time. It was found that this combination can be very useful in the management of cocaine addiction or dependence because combination of ondansetron and flumazenil markedly improves results compared to use of ondansetron or flumazenil alone. The use of a combination of ondansetron and flumazenil is novel.

### Amphetamines

Interaction of the 5-HT₃ antagonist ondansetron and d-amphetamine was investigated in 10 healthy human volunteers. After the subjects were pretreated with placebo or ondansetron (0.15 mg/kg, i.v.), 5-h challenge tests with oral D-amphetamine (0.5 mg/kg) were performed. It was found that those subjects with robust activation-euphoria responses to D-amphetamine had attenuated responses after ondansetron pretreatment (Grady, Broocks *et al*. 1996). The effects of different doses of ondansetron (0.1, 0.5, 1, 2 mg/kg) administered intra-peritoneally were studied on amphetamine-induced hyperactivity and stereotypy in Wistar rats. Ondansetron administered 30 minutes prior to d-amphetamine significantly decreased the mean number of head dippings and crossings in the hole board test and decreased the average stereotypic score indicating a potential role for ondansetron in conditions with dopamine excess (Shankar, Karan *et al*. 2000). In a clinical study, amphetamine (15 mg orally) produced a significant decrease in self-ratings of hunger 2.5 h after administration. This effect was significantly attenuated by pre-treatment with ondansetron (12 mg orally over 24 h). These findings are consistent with animal studies suggesting that ondansetron can attenuate certain catecholamine-mediated behaviors produced by amphetamines (Silverstone, Johnson *et al*. 1992). The effect of ondansetron on the psychological and psychomotor changes induced by amphetamine in human volunteers was studied. Nine healthy males took part in this double-blind placebo-controlled balanced-crossover study. Pretreatment with ondansetron attenuated the effects of amphetamine on hunger and subjective state, but not on blood pressure or psychomotor performance tests (Silverstone, Oldman *et al*. 1992).

In contrast a study found that 5-HT₃ receptor antagonists tested including ondansetron did not antagonize the effects of amphetamine suggesting that 5-HT₃ receptors cannot modulate the effects of amphetamine (Moser 1992).

There is no study describing the use of a combination of ondansetron and flumazenil for the management of amphetamine addiction or dependence. The use of both ondansetron and flumazenil either in the form of a combined formulation or administered according to a protocol for the management of amphetamine addiction of dependence is proposed for the first time. It was found that this combination can be very useful in the management of amphetamine addiction or dependence because combination of ondansetron and flumazenil markedly improves results compared to use of ondansetron or flumazenil alone. The use of a combination of ondansetron and flumazenil is novel.

### Nicotine, marijuana, THC and other drugs of abuse

The US FDA has approved a limited number of treatments for alcohol, nicotine and opioid dependence; however, no treatments for other abused drugs such as marijuana, cocaine or methamphetamine are approved. A substantial number of patients abuse more than one drug concurrently, complicating the treatment of substance use disorder (SUD) and leaving clinicians with few FDA-approved drug options for their patients. In this era of evidence-based medicine, such patients are typically treated with therapeutically proven medicaments, but in ways that are outside the scope of a drug's original indication by the FDA (Kenna, Nielsen *et al*. 2007).

Inpatients who were not receiving medication, were either smokers or nonsmokers, and had alcohol dependence completed 2 iodine I 123-labeled iomazenil single-photon emission computed tomographic scans to determine adaptations in gamma-aminobutyric acid type A (GABA(A))-benzodiazepine receptors. It was found that a time-dependent regulation of cortical GABA(A)-benzodiazepine receptors associated with the recovery from alcohol dependence occurred. Higher GABA(A)-benzodiazepine receptor levels during acute withdrawal may reflect a compensation for reduced receptor function, which is thought to contribute to alcohol tolerance and withdrawal (Staley, Gottschalk *et al*. 2005).

The ability of the selective 5-HT₃ receptor antagonist ondansetron to influence the behavioral consequences of withdrawal from chronic treatment with ethanol, nicotine or cocaine was investigated and it was found that ondansetron is a highly effective inhibitor of the increased behavioral suppression following withdrawal from the drugs of abuse like ethanol, nicotine and cocaine (Costall, Jones *et al*. 1990) and that 5-HT projections from the dorsal raphe nucleus may be involved in such behavior (Costall, Jones *et al*. 1990).

Ondansetron had no effect on self-stimulation, nor did it affect facilitation of responding by d-ampkaetamine. However, ondansetron reduced the initial depression of self-stimulation by high-dose nicotine, but not the ensuing facilitation. These results suggest that 5-HT₃ receptors mediate the excitatory effect of nicotine (Montgomery, Rose *et al*. 1993).

There is no study describing the use of a combination of ondansetron and flumazenil for the management of cannabis (marijuana, THC), nicotine (smoking) addiction or dependence. The use of both ondansetron and flumazenil either in the form a combined formulation or administered according to a protocol for the management of cannabis (marijuana, THC), nicotine (smoking) addiction or dependence is proposed for the first time. It was found that this combination can be very useful in the management of cannabis (marijuana, THC), nicotine (smoking) addiction or dependence because combination of ondansetron and flumazenil markedly improves results compared to use of ondansetron or flumazenil alone. The use of a combination of ondansetron and flumazenil is novel.

### Depression, mood and emotional disorders and loss of memory

Cholinergic receptor blockade produces memory deficits in animal models. There is evidence to suggest that serotonin (5-HT) plays an important role in learning and memory processes (Buhot, Martin *et al*. 2000). Studies have shown a functional interaction between serotonergic and cholinergic systems (Maura, Andrioli *et al*. 1992; Cassel and Jeltsch 1995) and it is possible that serotonergic receptors could modulate the activity of cholinergic system to cooperate in the regulation of cognitive processes (Barnes, Barnes *et al*. 1989; Cassel and Jeltsch 1995). The involvement of 5-HT₃ receptors in learning and memory has been repeatedly suggested and 5-HT₃ receptor antagonists, such as ondansetron, have been described as potential cognitive enhances in the treatment of dementia (Costal/ and Naylor 1997; Meneses 1998).

Therefore, these deficits can be prevented by 5-HT₃ receptor antagonists, such as ondansetron, which increases acetylcholine release. The effects on cognitive performance of combined treatments of ondansetron with either flumazenil, a GABA(A) receptor benzodiazepine site antagonist, or tacrine, a cholinesterase inhibitor, on scopolamine-induced cognitive impairment was investigated. The scopolamine-induced impairment of learning and retention in the water maze is fully prevented by ondansetron when given in combination with either flumazenil or tacrine, suggesting that both combined treatments may constitute the basis of a new therapy for cognitive disorders (Diez-Ariza, Redondo *et al*. 2003). However, there is no report of any preparation of such combination. It is not known whether it is possible to combine the two drugs to make a formulation that can be useful for the treatment of cognitive disorders.

GABA(A) receptor antagonists potentiated the effect induced by ondansetron on acetylcholine release, a peak increase of 238% with bicuculline, and 259% with flumazenil over basal levels was observed, suggesting an interaction of ondansetron with GABAergic neurons in modulating acetylcholine release in the rat frontal cortex (Diez-Ariza, Garcia-Alloza *et al*. 2002).

The 5-HT₃ receptor antagonists, ondansetron, MDL 72222 and granisetron produced a concentration-dependent increase of K+-evoked [3H]ACh efflux in slices from rat entorhinal cortex preloaded with [3H]choline. Bicuculline and flumazenil also enhanced [3H]ACh efflux (Diez-Ariza, Ramirez *et al*. 1998).

The combined ondansetron (0.1 microgram/kg) + flumazenil (10 mg/kg) administration significantly increased ACh release to a similar extent as a depolarising stimulus with K(+), 100 mM, suggesting that a combined ondansetron + flumazenil treatment can restore a diminished cholinergic function and may provide a basis for using this treatment in the therapy of cognitive disorders (Gil-Bea, Dominguez *et al*. 2004).

Muscarinic acetylcholine receptor antagonist scopolamine has been shown to selectively impair the accuracy of allocentric place discrimination task without changing swimming speed, distance, and still time. In this model neither flumazenil 10 mg/kg, ondansetron 0.3 mg/kg nor R(-)-alpha-metylhistamine 10 mg/kg attenuated the scopolamine-induced deficits (Kikusui, Tonohiro *et al*. 2000).

There is no study describing the use of a combination of ondansetron and flumazenil for the management of central nervous system disorders such as depression, mood and emotional disorders, loss of memory and the like. The use of both ondansetron and flumazenil either in the form a combined formulation or administered according to a protocol for the management of these disorders is proposed for the first time. It was found that this combination can be very useful in the management of CNS disorders because combination of ondansetron and flumazenil markedly improves results compared to use of ondansetron or flumazenil alone. The use of a combination of Ondansetron and flumazenil is novel.

These and other aspects and attributes of the present invention will be discussed with reference to the following description of the invention.

### Summary of the Invention

The present invention faces the problem of developing a method and a medicament for treatment of drug addiction or dependence or for treatment of a patient with a central nervous system (CNS) disorder.

Therefore, the object of the present invention is the use and methods of using a combination comprising a serotonin₃ (5-HT₃) receptor antagonist and a selective chloride channel modulator for treating a patient with drug addiction or dependence or for treatment of a patient with a central nervous system (CNS) disorder. In an embodiment, the method comprises administering to the patient an effective amount of the combination. An example of a 5-HT₃ receptor antagonist is ondansetron, and the selective chloride channel modulator is flumazenil. The drug can be a prescription drug or an illegal drug. Examples of the drug include but are not limited to alcohol, cocaine, nicotine, marijuana, benzodiazepines and opiates. Examples of CNS disorder include but are not limited to depression, mood and emotional disorders and loss of memory. The 5-HT₃ receptor antagonist and the selective chloride channel modulator may be administered together as a single-unit dose, or they may be administered separately as multi-unit doses wherein the 5-HT₃ receptor antagonist is administered before the selective chloride channel modulator or vice versa. In another embodiment, one or more than one dose of the 5-HT₃ receptor antagonist or one or more than one dose of the selective chloride channel modulator is administered. Preferably, the administration of the combination is parenteral.

In another embodiment, the method comprises administering to the patient an effective amount of a combination of ondansetron and flumazenil in a single formulation. The ratio of ondansetron to flumazenil may be from 1:1 to 100:1, or preferably from 2:1 to 20:1. The amount of ondansetron may be from 1 mg to 8 mg per dose, preferably 4 mg per dose. The amount of flumazenil may be from 1 mg to 4 mg per dose, preferably 2 mg per dose.

The object of the present invention is also the use of a combination comprising a therapeutically effective amount of a serotonin₃ (5-HT₃) receptor antagonist and a therapeutically effective amount of a selective chloride channel modulator for preparing a medicament for the treatment of said disorders. An example of the 5-HT₃ receptor antagonist is ondansetron, and the selective chloride channel modulator is flumazenil. In an embodiment of the invention, the 5-HT₃ receptor antagonist and the selective chloride channel modulator may be administered together as a single-unit dose, or in another embodiment, they may be administered separately as multi-unit doses wherein the 5-HT₃ receptor antagonist is administered before the selective chloride channel modulator or vice versa. In another possible embodiment, one or more than one dose of the 5-HT₃ receptor antagonist or one or more than one dose of the selective chloride channel modulator is administered. Preferably, the administration of the combination is parenteral.

### Detailed Description of the Invention

While the present invention is described in connection with what is presently considered to be the most practical and preferred embodiments, it should be appreciated that the invention is not limited to the disclosed embodiments, and is intended to cover various modifications and equivalent arrangements included within the spirit and scope of the claims. Modifications and variations in the present invention may be made without departing from the novel aspects of the invention as defined in the claims. The appended claims should be construed broadly and in a manner consistent with the spirit and the scope of the invention herein. It is understood that, given the above description of the embodiments of the invention, various modifications may be made by a person skilled in the art. Such modifications are intended to be encompassed by the claims below.

The present invention describes the use and methods of using a combination of a 5-HT₃ receptor antagonist, like ondansetron, with a selective chloride channel modulator, such as flumazenil, in definite concentrations corresponding to a single unit/mult-unit dose injectable preparation administered according to a defined protocol. The methods of administration described in the present invention are used for the treatment of patients with drug addiction or dependence. Drugs include prescription drugs as well as illegal drugs. Examples of drugs include but are not limited to alcohol, cocaine, nicotine, marijuana, benzodiazepines and opiates. In addition, the methods and compositions can be used for treatment of patients suffering from CNS disorders such as, but not limited to, depression, mood and emotional disorders and loss of memory.

There are procedures and protocols developed for the treatment of opioid addiction. A method not using pharmacological agent has been described in united States Patent Serial No. 6,368,111 entitled "System and method for interactively simulating and discouraging drug use" which relates to a system and method for interactively simulating and discouraging drug taking behavior, in which a simulated ingestion of drugs provides an initial enhanced sensation of movement and sound, and apparent enhanced player performance. The invention is illustrated by an implementation in the form of a modified multimedia motorcycle racing game. Innovation in pharmacological treatment of substance use disorders is progressing rapidly and United States Patent Serial No. 5,783,583 entitled "Improvements to rapid opioid detoxification" describes rapid opioid detoxification procedures which include administering an anesthetic agent and then an opioid antagonist while sedated. The method also describes administering a diarrhea suppressant, such as octreotide acetate. Another United States Patent Serial. No. 5,922,705 entitled "Rapid narcotic detoxification" describes methods for rapid detoxification of patients addicted to opioid narcotics. The methods include administering nalmefene to induce acute withdrawal, and administering dextromethorphan with nalmefene or other opioid antagonists to reduce the patient's subjective feelings of residual withdrawal symptoms following detoxification.

A United States Patent Serial No- 5,783,583 entitled "17-(cyclypropylmethyl)-4,5alpha-epyxy-6-methylenemorphinan-3,14-diol, hydrochloride salt for the purpose of rapid narcotic detoxification" relates to novel methods using a chemical compound which possesses special characteristics that makes it uniquely ideal for active and rapid medical detoxification of human beings addicted to exogenous narcotic drugs. The general classification of said chemical compound is known as narcotic antagonists. The chemical compound 17(cycloprapylmethyl)-4,5alpha-epoxy-6-methylenemorphinan-3,14-diol,hydrochloride salt, known as nalmefene, may be used for detoxifying human beings addicted to narcotics in association with anesthesia because it is able to be administered intravenously, and because it does not cause hepatocellular injury as does naltrexone. On similar lines a United States Patent 6,103,734 entitled "Drug combination as a medicament to suppress the dependence of individuals to opiates" relates to a combination of chemical compounds used as a medicament intended to suppress the dependence of individuals to opioids, the combination comprising a laxative or enema of irrigation, alpha-adrenergic agents, anti-emetic agents, gastric protectors, anxiolytic compound, anesthetic sleep inducing agent and an opioid antagonist compound, such as nazalone or naltrexone.

Recent advances in the treatment of cocaine addition has been described in United States Patent 7,186,711 entitled "Flumazenil for the treatment of cocaine dependency" where it has been described that flumazenil can be administered sequentially in small amounts at short intervals for the treatment of cocaine dependency. Similarly, the use of flumazenil in treatment of alcohol dependency has been described in United States Patent Application 20040092509 entitled "Use of flumazenil in the production of a drug for the treatment of alcohol dependency" where flumazenil has been proposed to be administered sequentially in small amounts at short intervals for the treatment of alcohol dependency. Furthermore, another United States Patent Application 20050192271 entitled "Use of selective chloride channel modulators to treat alcohol and/or stimulant substance abuse" describes the use of pharmaceutical compositions from a class of compounds that directly or indirectly selectively modulate GABA.sub.A chloride channel activity using a substance such as flumazenil to treat alcohol and/or stimulant substance abuse.

The present invention is the first to use a combination of a 5-HT₃ receptor antagonist such as flumazenil and a benzodiazepine receptor antagonist such as ondansetron for the treatment of patients with drug addiction or dependence. In addition, the preparation can be used for treatment of patients suffering from CNS disorders such as depression, mood and emotional disorders, loss of memory and the like.

Therefore, the present invention discloses the use and methods, of using a combination comprising a serotonin₃ (5-HT₃) receptor antagonist and a selective chloride channel modulator for treating a patient with drug addiction or dependence or for treatment of a patient with a central nervous system (CNS) disorder. In an embodiment, the method comprises administering to the patient an effective amount of the combination. An example of a 5-HT₃ receptor antagonist is ondansetron, and the selective chloride channel modulator is flumazenil. The drug can be a prescription drug or an illegal drug. Examples of the drug include but are not limited to alcohol, cocaine, nicotine, marijuana, benzodiazepines and opiates. Examples of the CNS disorder include but are not limited to depression, mood and emotional disorders and loss of memory. The 5-HT₃ receptor antagonist and the selective chloride channel modulator may be administered together as a single-unit dose, or they may be administered separately as multi-unit doses wherein the 5-HT₃ receptor antagonist is administered before the selective chloride channel modulator, such as for example 4, 3, 2, 1 or half an hour before or even immediately before, or vice versa. In another embodiment, one or more than one dose of the 5-HT₃ receptor antagonist or one or more than one dose of the selective chloride channel modulator is administered. Preferably, the administration of the combination is parenteral.

In another embodiment, the method comprises administering to the patient an effective amount of a combination of ondansetron and flumazenil in a single formulation. The ratio of ondansetron to flumazenil may be from 1:1 to 100:1, or preferably from 2:1 to 20:1. The amount of ondansetron may be from 1 mg to 8 mg per dose, or may preferably be 4 mg per dose. The amount of flumazenil may be from 1 mg to 4 mg per dose, or may preferably be 2 mg per dose.

The present invention also relates to the use of a combination comprising a therapeutically effective amount of a serotonin₃ (5-HT₃) receptor antagonist, preferably ondansetron, and a therapeutically effective amount of a selective chloride channel modulator, preferably flumazenil, for preparing a medicament for treatment of drug addiction or dependence or for treatment of a central nervous system (CNS) disorder.

The drug in the drug addiction or dependence is a prescription drug or an illegal drug, such as for example alcohol, cocaine, nicotine, marijuana, benzodiazepines and opiates. Examples of the CNS disorder include but are not limited to depression, mood and emotional disorders and loss of memory.

In a preferred embodiment, the 5-HT₃ receptor antagonist and the selective chloride channel modulator may be administered together as a single-unit dose.

In another preferred embodiment, the 5-HT₃ receptor antagonist and the selective chloride channel modulator may be administered separately as a multi-unit doses. In this embodiment, the 5-HT₂ receptor antagonist may be administered before the selective chloride channel modulator, such as for example 4, 3, 2, 1 or half an hour before or even immediately before, or vice versa.

In a particular embodiment, one or more than one dose of the 5-HT₃ receptor antagonist may be administered or one or more than one dose of the selective chloride channel modulator may be administered. construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (for example, "such as") provided herein, is intended merely to better illuminate the invention and does not pose a limitation on the scope of the invention unless otherwise claimed. No language in the specification should be construed as indicating any non-claimed element as essential to the practice of the invention.

### Examples

The following examples illustrate the invention and must not be construed as limiting the scope thereof.

Before starting the treatment, the patients were subjected to a complete medical and psychological examination, including the measurement of depression such as BDI II and SCL 90. The monitoring of the patients included a thorough blood analysis with a complete count, a biochemical profile (creatinine, glucose, urea, HDL and LDL cholesterol, triglycerides, alkaline phosphatase, LDH (lactate dehydrogenase) and total proteins), liver function tests (GOT, GPT, GGT, bilirubin), electrocardiogram and, if necessary, pregnancy test and X-ray examination. The exclusion criteria included acute or uncompensated diseases.

The heart rate and the blood pressure were assessed before and after the administration of the combination.

The combination was administered intravenously, for example, by means of boluses containing the suitable amount and observing the reaction in the patient.

In the event that the patient has a panic attack, a suitable therapeutic agent, for example, hydroxyzine hydrochloride or clomethiazole, may be administered before the administration of the combination.

Sequence of administration of a standard drug for treatment of depression,

| Time | Day of | Day 1 | Day 2 | Day 3 | Day of |
|---|---|---|---|---|---|
| | admission | | | | discharge |
| 9:00 | Hydroxyzine | Hydroxyzine | Hydroxyzine | Hydroxyzine | Hydroxyzine |
| | hydrochloride | hydrochloride | hydrochloride | hydrochloride | hydrochloride |
| | 25-50 mg | 25-50 mg | 25-50 mg | 25-50 mg | 25-50 mg |
| 11:00 | Ondansetron | Ondansetron | Ondansetron | Ondansetron | Ondasetron |
| | 4 mg i.v. | 4 mg i.v | 4 mg i.v | 4 mg i.v | 4 mg i.v |
| 11:30 | Flumazenil | Flumazenil | Flumazenil | Flumazenil | Flumazenil |
| | 2 mg i.v. | 2 mg i.v. | 2 mg i.v. | 2 mg i.v. | 2 mg i.v. |
| 21:30 | Hydroxyzine | Hydroxyzine | Hydroxyzine | Hydroxyzine | Hydroxyzine |
| | hydrochloride | hydrochloride | hydrochloride | hydrochloride | hydrochloride |
| | 25-50 mg | 25-50 mg | 25-50 mg | 25-50 mg | 25-50 mg |

Note: If it is not possible to use hydroxyzine hydrochloride, it can be substituted with clomethiazole in the approximate amount of 192 mg.

Flumazenil should be administered by means of a continuous intravenous perfusion of 100 cc of saline solution in a period of 45 minutes.

The intravenous administration of flumazenil, to a total dose of 2.0 mg, would start with a 0.1 mg bolus every 3 minutes. After two administrations, this dose would be increased to 0.2 mg. If there is no exhaustion or suffering of any type reported by the patient or observed by the doctor after the second bolus, under these conditions, i.e., no suffering observed or reported, the bolus could be increased to 0.3 mg and the administration could decrease to 2 minutes between boluses. If suffering of any type is observed or reported upon increasing the dose or decreasing the time between boluses, the administration should continue at the previous level of non-suffering, or return to the initial level.

The administration parameters of flumazenil could be

Preferably, said medicament is administered parenterally.

The ratio of ondansetron to flumazenil in the combination according to the invention is from 1:1 to 100:1, preferably from 2:1 to 20:1.

The therapeutically effective amount of ondansetron in the combination according to the invention is from 1 mg to 8 mg per dose, it is preferably 4 mg per dose.

The therapeutically effective amount of ondansetron in the combination according to the invention is from 1 mg to 4 mg per dose, it is preferably 2 mg per dose.

The combination may be administered sequentially, at short time intervals, in small amounts until administering a therapeutically effective amount.

More specifically, the invention relates to the use of said combination for preparing the medicament for sequential administration, at time intervals comprised between 1 and 15 minutes, of amounts of flumazenil and ondansetron comprised between 0.1 and 0.3 mg, until administering a therapeutically effective amount.

As used herein, the term "effective amount" refers to an amount of both the active ingredients that is sufficient, when administered by means of the method of the invention, to differentially deliver suitable agents which may be used for the treatment of alcohol, cocaine, nicotine, marijuana, benzodiazepines and opiate addicted and dependent patients. Furthermore, the preparation can be used for treatment of patients suffering from depression, mood and emotional disorders and loss of memory.

The term "parenteral administration" herein embraces the means of injection or infusion of a composition into veins that is intravenously only.

The use of the terms "a" and "an" and "the" and similar referents in the context of describing the invention (especially in the context of the following claims) are to be modified in some cases without harmful effects in the treatment. The amounts may be greater than 0.2 mg, the bolus could be greater than 0.3 mg and the period could be increased or decreased by a few minutes. Nevertheless, the parameters established in this protocol are the suitable ones in all the treated cases to induce minimum or zero exhaustion and discomfort, facilitating the recovery and the absence of withdrawal.

The intravenous treatment should be administered without exhaustion or suffering being reported by the patient or observed by the doctor. If exhaustion is observed or reported upon increasing the doses or decreasing the time between doses, the administration should continue at the previous level of non-suffering or return to the initial level.

Ondansetron 4 mg should be infused intravenously every 15 minutes and administered essentially 30 minutes before flumazenil.

### Experimental protocols

### Example 1

Age - 35 years
Sex - Male

This 35-year-old patient was admitted with complaints of loss of pleasure in all activities and sleep disorder with early awakening in the morning.

| Time | Day of | Day 1 | Day 2 | Day 3 | Day of |
|---|---|---|---|---|---|
| | admission | | | | discharge |
| 9:00 | Hydroxyzine | Hydroxyzine | Hydroxyzine | Hydroxyzine | Hydroxyzine |
| | hydrochloride | hydrochloride | hydrochloride | hydrochloride | hydrochloride |
| | 25 mg | 25 mg | 25 mg | 25 mg | 25 mg |
| 11:00 | Ondansetron | Ondansetron | Ondansetron | Ondansetron | Ondansetron |
| | 4 mg i.v. | 4 mg i.v | 4 mg i.v | 4 mg i.v | 4 mg i.v |
| 11:30 | Flumazenil | Flumazenil | Flumazenil | Flumazenil | Flumazenil |
| | 2 mg i.v. | 2 mg i.v. | 2 mg i.v. | 2 mg i.v. | 2 mg i.v. |
| 21:30 | Hydroxyzine | Hydroxyzine | Hydroxyzine | Hydroxyzine | Hydroxyzinc |
| | hydrochloride | hydrochloride | hydrochloride | hydrochloride | hydrochloride |
| | 25 mg | 25 mg | 25 mg | 25 mg | 25 mg |

The rate of infusion of the drug was maintained at 0.3 mg every 3 minutes for the entire duration of the study.

There were no side effects during the total treatment period.

### Example 2

Age - 46 years
Sex - Male

This 46-year-old patient was admitted with complaints of fatigue, loss of appetite, with sleep disorder.

| Time | Day of admission | Day 1 | Day 2 | Day 3 | Day of discharge |
|---|---|---|---|---|---|
| 8:30 | Hydroxyzine hydrochloride 25 mg | Hydroxyzine hydrochloride 25 mg | Hydroxyzine hydrochloride 25 mg | Hydroxyzine hydrochloride 25 mg | Hydroxyzine hydrochloride 25 mg |
| 10:30 | Ondansetron 4 mg i.v. | Ondansetron 4 mg i.v | Ondansetron 4 mg i.v | Ondansetron 4 mg i.v | Ondansetron 4 mg i.v |
| 11:00 | Flumazenil 2 mg i.v. | Flumazenil 2 mg i.v. | Flumazenil 2 mg i.v. | Flumazenil 2 mg i.v. | Flumazenil 2 mg i.v. |
| 21:00 | Hydroxyzine hydrochloride 25 mg | Hydroxyzine hydrochloride 25 mg | Hydroxyzine hydrochloride 25 mg | Hydroxyzine hydrochloride 25 mg | Hydroxyzine hydrochloride 25 mg |

The rate of infusion of the drug was maintained at 0.3 mg every 3 minutes for the entire duration of the study.

There were no side effects during the total treatment period.

### Example 3

Age - 48 years
Sex - Male

This 48-year-old patient was admitted with complaints of loss of concentration in activities with irritability.

| Time | Day of admission | Day 1 | Day 2 | Day 3 | Day of discharge |
|---|---|---|---|---|---|
| 8:30 | Hydroxyzine hydrochloride 25 mg | Hydroxyzine hydrochloride 25 mg | Hydroxyzine hydrochloride 25 mg | Hydroxyzine hydrochloride 25 mg | Hydroxyzine hydrochloride 25 mg |
| 10:00 | Ondansetron 4 mg i.v. | Ondansetron 4 mg i.v | Ondansetron 4 mg i.v | Ondansetron 4 mg i.v | Ondansetron 4 mg i.v |
| 10:30 | Flumazenil 2 mg i.v. | Flumazenil 2 mg i.v. | Flumazenil 2 mg i.v. | Flumazenil 2 mg i.v. | Flumazenil. 2 mg i.v. |
| 20:30 | Hydroxyzine hydrochloride 25 mg | Hydroxyzine hydrochloride 25 mg | Hydroxyzine hydrochloride 25 mg | Hydroxyzine hydrochloride 25 mg | Hydroxyzine hydrochloride 25 mg |

The rate of infusion of the drug was maintained at 0.3 mg every 3 minutes for the entire duration of the study.

There were no side effects during the total treatment period.

### Example 4

Age - 42 years
Sex - Female

This 42-year-old patient was admitted with complaints of loss of sleep, appetite and irritability.

| Time | Day of admission | Day 1 | Day 2 | Day 3 | Day of discharge |
|---|---|---|---|---|---|
| 8:45 | Hydroxyzine hydrochloride 25 mg | Hydroxyzine hydrochloride 25 mg | Hydroxyzine hydrochloride 25 mg | hydroxyzine hydrochloride 25 mg | Hydroxyzine hydrochloride 25 mg |
| 10:45 | Ondansetron 4 mg i.v. | Ondansetron 4 mg i.v | Ondansetron 4 mg i.v | Ondansetron 4 mg i.v | Ondansetron 4 mg i.v |
| 11:30 | Flumazenil 2 mg i.v. | Flumazenil 2 mg i.v. | Flumazenil 2 mg i.v. | Flumazenil 2 mg i.v. | Flumazenil 2 mg i.v. |
| 21:45 | Hydroxyzine hydrochloride 25 mg | Hydroxyzine hydrochloride 25 mg | Hydroxyzine hydrochloride 25 mg | Hydroxyzine hydrochloride 25 mg | Hydroxyzine hydrochloride 25 mg |

The rate of infusion of the drug was maintained at 0.3 mg every 3 minutes for the entire duration of the study.

There were no side effects during the total treatment period.

### Example 5

Age - 34 years
Sex - Female

This 34-year-old patient was admitted with complaints of loss of pleasure in all activities and sleep disorder.

| Time | Day of admission | Day 1 | Day 2 | Day 3 | Day of discharge |
|---|---|---|---|---|---|
| 8:00 | Hydroxyzine hydrochloride 25 mg | Hydroxyzine hydrochloride 25 mg | Hyoxyzine hydrochloride 25 mg | Hydroxyzine hydrochloride 25 mg | Hydroxyzine hydrochloride 25 mg |
| 10:00 | Ondansetron 4 mg i.v. | Ondansetron 4 mg i.v | Ondansetron 4 mg i.v | Ondansetron 4 mg i.v | Ondansetron 4 mg i.v |
| 10:30 | Flumazenil 2 mg i.v. | Flumazenil 2 mg i.v. | Flumazenil 2 mg i.v. | Flumazenil 2 mg i.v. | Flumazenil 2 mg i.v. |
| 20:30 | Hydroxyzine hydrochloride 25 mg | Hydroxyzine hydrochloride 25 mg | Hydroxyzine hydrochloride 25 mg | Hydroxyzine hydrochloride 25 mg | Hydroxyzine hydrochloride 25 mg |

The rate of infusion of the drug was maintained at 0.2 mg every 3 minutes for the entire duration of the study.

Only anxiety was presented as a side effect during the total treatment period.

### Example 6

Age - 48 years
Sex - Male

This 48-year-old patient was admitted with complaints of irritability with loss of concentration in all activities and sleep disorder with early awakening in the morning.

| Time | Day of admission | Day 1 | Day 2 | Day 3 | Day of discharge |
|---|---|---|---|---|---|
| 8:30 | Hydroxyzine hydrochloride 25 mg | Hydroxyzine hydrochloride 25 mg | Hydroxyzine hydrochloride 25 mg | Hydroxyzine hydrochloride 25 mg | Hydroxyzine hydrochloride 25 mg |
| 10:30 | Ondansetron 4 mg i.v. | Ondansetron 4 mg i.v | Ondansetron 4 mg i.v | Ondansetron 4 mg i.v | Ondansetron 4 mg i.v |
| 11:30 | Flumazenil 2 mg i.v. | Flumazenil 2 mg i.v. | Flumazenil 2 mg i.v. | Flumazenil 2 mg i.v. | Flumazenil 2 mg i.v. |
| 21:00 | Hydroxyzine hydrochlorid. 25 mg | Hydroxyzine hydrochloride 25 mg | Hydroxyzine hydrochloride 25 mg | Hydroxyzine hydrochloride 25 mg | Hydroxyzine hydrochloride 25 mg |

The rate of infusion of the drug was maintained at 0.2 mg every 3 minutes for the entire duration of the study.

There were no side effects during the total treatment period.

### References

Ait-Daoud, N., B. A. Johnson, et al. (2001). "Combining ondansetron and naltrexone reduces craving among biologically predisposed alcoholics: preliminary clinical evidence." Psychopharmacology (Berl) 154(1): 23-27.
Artaiz, I., G. Romero, et al. (1995). "The pharmacology of VA21B7: an atypical 5-HT3 receptor antagonist with anxiolytic-like properties in animal models." Psychopharmacology (Berl) 117(2) : 137-148.
Barnes, J. M., N. M. Barnes, et al. (1989). "5-HT3 receptors mediate inhibition of acetylcholine release in cortical tissue." Nature 338(6218): 762-3.
Biggio, F., G. Gorini, et al. (2007). "Flumazenil selectively prevents the increase in alpha(4)-subupit gene expression and an associated change in GABA(A) receptor function induced by ethanol withdrawal." J Neurochem.
Borisenko, S. A., Q. H. Meng, et al. (1996). "Neurochemical mediators of anxiety nave inconsistent effects on hypothalamic self-stimulation in rats." Pharmacol Toxicol 78(5): 354-360.
Buhot, M. C, S. Martin, et al. (2000). "Role of serotonin in memory impairment." Ann Med 32(3) : 210-21.
Burton, J. H., L. Lyon, et al. (1998). "Continuous flumazenil infusion in the treatment of zolpidem (Ambien) and ethanol coingestion." J Toxicol Clin Toxicol 36(7): 743-746.
Cassel, J. C. and H. Jeltsch (1995). "Serotonergic modulation of cholinergic function in the central nervous system: cognitive implications." Neuroscience 69(1): 1-41.
Collins, G. B., M. S. McAllister, et al. (2006). "Drug adjuncts for treating alcohol dependence." Cleve Clin J Med 73 (7) : 641-644.
Costall, B., B. J. Jones, et al. (1989). "The effects of ondansetron, (GR38032F) in rats and mice treated subchronically with diazepam." Pharmacol Biochem Behav 34(4): 769-778.
Costall, B., B. J. Jones, et al: (1990). "Ondansetron inhibits a behavioural consequence of withdrawing from drugs of abuse." Pharmacol Biochem Behav 36(2): 339-344.
Costall, B., B. J. Jones, et al. (1990). "Sites of action of ondansetron to inhibit withdrawal from drugs of abuse." Pharmacol Biochem Behav 36(1): 97-9104.
Costall, B. and R. J. Naylor (1997). "The influence of 5-HT2 and 5-HT4 receptor antagonists to modify drug induced disinhibitory effects in the mouse light/dark test." Br J Pharmacol 122(6): 1105-18.
Davidson, C, C. Lazarus, et al. (2004). "Ondansetron, given during the acute cocaine withdrawal, attenuates oral cocaine self-administration." Eur J Pharmacol 503(1-3): 99-9102.
Davidson, C, T. H. Lee, et al. (2002). "Ondansetron given in the acute withdrawal from a repeated cocaine sensitization dosing regimen reverses the expression of sensitization and inhibits self-administration." Neuropsychopharmacology 27(4): 542-553.
Dawes, M. A., B. A. Johnson, et al. (2005). "A prospective, open-label trial of ondansetron in adolescents with alcohol dependence." Addict Behav 30(6): 1077-1085.
Dawes, M. A., B. A. Johnson, et al. (2005). "Reductions in and relations between "craving" and drinking in a prospective, open-label trial of ondansetron in adolescents with alcohol dependence." Addict Behav 30(9): 1630-1637.
Derlet, R. W. and T. E. Albertson (1994). "Flumazenil induces seizures and death in mixed cocaine-diazepam intoxications." Ann Emery Med 23(3): 494-498.
Diez-Ariza, M., M. Garcia-Alloza, et al. (2002)-"GABA(A) receptor antagonists enhance cortical acetylcholine release induced by 5-HT(3) receptor blockade in freely moving rats." Brain Res 956(1): 81-85.
Diez-Ariza, M., M. J. Ramirez, et al. (1998)-"Differential interaction between 5-HT3 receptors and GABAergic neurons inhibiting acetylcholine release in rat entorhinal cortex slices." Brain Res 801(1-2): 228-232.
Diez-Ariza, M., C. Redondo, et al. (2003). "Flumazenil and tacrine increase the effectiveness of ondansetron on scopolamine-induced impairment of spatial learning in rats." Psychopharmacology (Berl) 169(1): 35-41.
Dooley, D. J. and I. Klamt (1993). "Differential profile of the CCKB receptor antagonist CI-988 and diazepam in the four-plate test." Psychopharmacology (Berl) 112(4): 452-454.
Gil-Bea, F. J., J. Dominguez, et al. (2004). "Facilitation of cholinergic transmission by combined treatment of ondansetron with flumazenil after cortical cholinergic deafferentation." Neuropharmacology 47(2): 225-232.
Grady, T. A., A. Broocks, et al. (1996). "Biological and behavioral responses to D-amphetamine, alone and in combination with the serotonins receptor antagonist ondansetron, in healthy volunteers." Psychiatry Res 64(1): 1-10.
Heilig, M. and M. Egli (2006). "Pharmacological treatment of alcohol dependence: target symptoms and target mechanisms." Pharmacol Ther 111(3): 855-876.
Johnson, B. A., N. Ait-Daoud, et al. (2003). "Ondansetron reduces mood disturbance among biologically predisposed, alcohol-dependent individuals." Alcohol Clin Exp Res 27(11): 1773-1773.
Johnson, B. A., N. Ait-Daoud, et al. (2000). "Combining ondansetron and naltrexone effectively treats biologically predisposed alcoholics: from hypotheses to preliminary clinical evidence." Alcohol Clin Exp Res 24(5): 737-742.
Johnson, B. A., J. D. Roache, et al. (2006). "A preliminary randomized, double-blind, placebo-controlled study of the safety and efficacy of ondansetron in the treatment of cocaine dependence." Drug Alcohol Depend 84(3): 256-263.
Johnson, B. A., J. D. Roache, et al. (2002). "Ondansetron reduces the craving of biologically predisposed alcoholics." Psychopharmacology (Berl) 160(4): 408-413.
June, H. L., D. Zuccarelli, et al. (1998). "High-affinity benzodiazepine antagonists reduce responding maintained by ethanol presentation in ethanol-preferring rats." J Pharmacol Exp Ther 284(3): 1006-1014.
Kenna, G. A., D. M. Nielsen, et al. (2007). "Pharmacotherapy of dual substance abuse and dependence." CNS Drugs 21(3) : 213-237.
Kikusui, T., T. Tonohiro, et al. (2000). "The allocentric place discrimination task is selectively and highly dependent on the central muscarinic system in rats." Pharmacol Biochem Behav 65(1) : 131-139.
King, G. R., Z. Xiong, et al. (1997). "Blockade of cocaine sensitization and tolerance by the co-administration of ondansetron, a 5-HT3 receptor antagonist, and cocaine." Psychopharmacology (Berl) 130(2): 159-165.
King, G. R., Z. Xiong, et al. (1998). "Blockade of the expression of sensitization and tolerance by ondansetron, a 5-HT3 receptor antagonist, administered during withdrawal from intermittent and continuous cocaine." Psychopharmacology (Berl) 135 (3) : 263-269.
Knapp, D. J., D. H. Overstreet, et al. (2004). "SB242084, flumazenil, and CRA1000 block ethanol withdrawal-induced anxiety in rats." Alcohol 32(2): 101-111.
Lingford-Hughes, A. R., S. J. Wilson, efc to the. (2005). "GABA- benzodiazepine receptor function in alcohol dependence: a combined HC-flumazenil PET and pharmacodynamic study." Psychopharmacology (Berl) 180(4): 595-606.
Maura. G., G. C. Andrioli, et al. (1992). "5-Hydroxytryptamine3 receptors sited on cholinergic axon terminals of human cerebral cortex mediate inhibition of acetylcholine release." J Neurochem 58(6): 2334-7.
Meneses, A. (1998). "Physiological, pathophysiological and therapeutic roles of 5-HT systems in learning and memory." Rev Neurosci 9(4): 275-89.
Montgomery, A. M., I. C. Rose, et al. (1993). "The effect of a 5-HT3 receptor antagonist, ondansetron, on brain stimulation reward, and its interaction with direct and indirect stimulants of central dopaminergic transmission." J Neural Transm Gen Sect 91(1) : 1-11.
Moser, P. C. (1992). "The effect of 5-HT3 receptor antagonists on the discriminative stimulus effects of amphetamine." Eur J Pharmacol 212(2-3): 271-274
Moy, S. S., D. J. Knapp, et al. (1997). "Flumazenil blockade of anxiety following ethanol withdrawal in rats." Psychopharmacology (Berl) 131(4): 354-360.
O'Brien, C. P. (1997). "A range of research-based pharmacotherapies for addiction." Science 278(5335): 66-70.
Potokar, J., N. Coupland, et al. (1997). "Flumazenil in alcohol withdrawal: a double-blind placebo-controlled study." Alcohol 32(5): 605-611.
Prather, P. L., S. M. Rezazadeh, et al. (1993). "Conflicting evidence regarding the efficacy of ondansetron in benzodiazepine withdrawal." J Pharmacol Exp Ther 264(2): 622-630.
Rezazadeh, S. M., P. L. Rather, et al. (1992) . "Evaluation of anxiolytic action of ondansetron, in rats during withdrawal from chronic chlordiazepoxide." Ann N Y Acad Sci 654: 472-473.
Roychoudhury, M. and S. K. Kulkarni (1997). "Antianxiety profile of ondansetron, a selective 5-HT3 antagonist, in a novel animal model." Methods Find Exp Clin Pharmacol 19(2): 107-111.
Shankar, R. P., R. S. Karan, et al. (2000). "Effect of the 5-HT3 receptor antagonist ondansetron on amphetamine-induced hyperactivity and stereotype in rats." Indian J Physiol Pharmacol 44(3): 355-358.
Silverstone, P. H., B. Johnson, et al. (1992). "Does ondansetron attenuate amphetamine-induced behaviour in human volunteers?" Psychopharmacology (Berl) 107(1): 140-141.
Silverstone, P. H., D. Oldman, et al. (1992). "Ondansetron, a 5-HT3 receptor antagonist, partially attenuates the effects of amphetamine : a pilot study in healthy volunteers." Int Clin Psychopharmacol 7(1): 37-43.
Staley, J. K., C. Gottschalk, et al. (2005). "Cortical gamma-aminobutyric acid type A-benzodiazepine receptors in recovery from alcohol dependence: relationship to features of alcohol dependence and cigarette smoking." Arch Gen Psychiatry 62 (8): 877-888.
Stanford, B. J. and S. C. Stanford (1999) "Postoperative delirium indicating an adverse drug interaction involving the selective serotonin reuptake inhibitor, paroxetine?" J Psychopharmacol 13(3): 313-317.
Trescot, A. M., M. V. Boswell, et al. (2006). "Opioid guidelines in the management of chronic non-cancer pain." Pain Physician 9(1): 1-39.

## Claims

1. A method for treating a patient with drug addiction or dependence or for treatment of a patient with a central nervous system (CNS) disorder, the method comprising administering to the patient a therapeutically effective amount of a combination comprising a serotonin, (5-HT₃) receptor antagonist and a selective chloride channel modulator.

2. The method according to claim 1, wherein the 5-HT₃ receptor antagonist is ondansetron, and the selective chloride channel modulator is flumazenil.

3. The method according to claim 1, wherein the drug in the drug addiction or dependence is a prescription drug or an illegal drug.

4. The method according to claim 1, wherein the drug in the drug addiction or dependence is selected from the group consisting of: alcohol, cocaine, amphetamine, crack, methamphetamine, nicotine, marijuana, benzodiazepines, methadone, codeine and opiates.

5. The method according to claim 1, wherein the CNS disorder is selected from the group consisting of: depression, mood and emotional disorders and loss of memory.

6. The method according to claim 1, wherein the 5-HT₃ receptor antagonist and the selective chloride channel modulator are administered together as a single-unit dose.

7. The method according to claim 1, wherein the 5-HT₃ receptor antagonist and the selective chloride channel modulator are administered separately as multi-unit doses.

8. The method according to claim 1, wherein the 5-HT₃ receptor antagonist is administered before the selective chloride channel modulator or vice versa.

9. The method according to claim 1, wherein one or more than one dose of the 5-HT₃ receptor antagonist is administered or one or more than one dose of the selective chloride channel modulator is administered.

10. The method according to claim 1, wherein the administration is parenteral.

11. A method for treating a patient with drug addiction or dependence or for treatment of a patient with a central nervous system (CNS) disorder, the method comprising administering a therapeutically effective amount of a combination of ondansetron and flumazenil in a single formulation to the patient.

12. The method according to claim 11, wherein the drug in the drug addiction or dependence is a prescription drug or an illegal drug.

13. The method according to claim 11, wherein the drug in the drug addiction or dependence is selected from the group consisting of: alcohol, cocaine, nicotine, marijuana, benzodiazepines and opiates.

14. The method according to claim 11, wherein the CNS disorder is selected from the group consisting of: depression, mood and emotional disorders and loss of memory.

15. The method according to claim 11, wherein the ratio of ondansetron to flumazenil is from 1:1 to 100:1.

16. The method according to claim 11, wherein the ratio of ondansetron to flumazenil is from 2:1 to 20:1

17. The method according to claim 11, wherein said therapeutically effective amount of ondansetron is from 1 mg to 8 mg per dose.

18. The method according to claim 11, wherein said therapeutically effective amount of ondansetron is 4 mg per dose.

19. The method according to claim 11, wherein said therapeutically effective amount of flumazenil is from 1 mg to 4 mg per dose.

20. The method according to claim 11, wherein said therapeutically effective amount of flumazenil is 2 mg per dose.

21. The method according to claim 20, wherein said therapeutically effective amount of flumazenil is from 0.1 mg per dose in intervals of 1 to 3 minutes until reaching an effective dose which is 2 mg/day in an average of 3 days.

22. Use of a combination comprising a therapeutically effective amount of a serotonin₃ (5-HT₃) receptor antagonist and a therapeutically effective amount of a selective chloride channel modulator for preparing a medicament for treatment of drug addiction or dependence or for treatment of a central nervous system (CNS) disorder.

23. Use according to claim 22, wherein the 5-HT₃ receptor antagonist is ondansetron, and the selective chloride channel modulator is flumazenil.

24. Use according to claim 22, wherein the drug in the drug addiction or dependence is a prescription drug or an illegal drug.

25. Use according to claim 24, wherein the drug in the drug addiction or dependence is selected from the group consisting of: alcohol, cocaine, nicotine, marijuana, benzodiazepines and opiates.

26. Use according to claim 22, wherein the CNS disorder is selected from the group consisting of: depression, mood and emotional disorders and loss of memory.

27. Use according to claim 22, wherein the 5-HT₃ receptor antagonist and the selective chloride channel modulator are administered together as a single-unit dose.

28. Use according to claim 22, wherein the 5-HT₃ receptor antagonist and the selective chloride channel modulator are administered separately as multi-unit doses.

29. Use according to claim 28, wherein the 5-HT₃ receptor antagonist is administered before the selective chloride channel modulator or vice versa -

30. Use according to claim 22 or 28, wherein one or more than one dose of the 5-HT₃ receptor antagonist is administered or one or more than one dose of the selective chloride channel modulator is administered.

31. Use according to claim 22, wherein said medicament is administered parenterally.

32. Use according to claim 23, wherein the ratio of ondansetron to flumazenil is from 1:1 to 100:1.

33. Use according to claim 32, wherein the ratio of ondansetron to flumazenil is from 2:1 to 20:1.

34. Use according to claim 33, wherein the ratio of ondansetron to flumazenil is from 1:1 to 10:1.

35. Use according to claim 23, wherein said therapeutically effective amount of ondansetron is from 1 mg to 8 mg per dose.

36. Use according to claim 35, wherein said therapeutically effective amount of ondansetron is 4 mg per dose.

37. Use according to claim 23, wherein said therapeutically effective amount of flumazenil is from 1 mg to 4 mg per dose.

38. Use according to claim 37, wherein said therapeutically effective amount of flumazenil is 2 mg per dose.

39. Use according to claim 38, wherein said therapeutically effective amount of flumazenil is from 0.1 mg per dose in intervals of 1 to 3 minutes until reaching an effective dose which is 2 mg/day in an average of 3 days.
